# EUROPÄISCHE PATENTANMELDUNG

(11) **EP 4 656 180 A1**
(43) Veröffentlichungstag der Anmeldung: **03.12.2025**
(21) Anmeldenummer: 24178513.8
(22) Anmeldetag: 28.05.2024
(51) Int. Cl.: A61K 9/00, A61K 9/51, A61K 47/54, A61K 47/69, A61P 5/00, A61P 31/02, A61P 35/00, A61P 37/02

(54) **NANOTEILCHEN ZUR VERABREICHUNG EINES WIRKSTOFFS**

(71) Anmelder: Cantreat AG, 8832 Wollerau (CH)
(72) Erfinder: BERNKOP-SCHNÜRCH, Andreas, 8832 Wollerau (CH); FORSTER, Michael, 6060 Hall in Tirol (AT); HOLD, Dietmar, 8810 Horgen (CH); STEINMÜLLER, Thomas, 6060 Hall in Tirol (AT); SUMMONTE, Simona, 8832 Wollerau (CH)
(74) Vertreter: Schwarz & Partner Patentanwälte GmbH

(57) **Zusammenfassung**

Nanoteilchen (1) zur Verabreichung zumindest eines Wirkstoffs (4), umfassend einen magnetisierbaren Kern (2), wobei der Kern (2) eine Ummantelung (3) aus einem Polyphosphat aufweist, wobei die Ummantelung (3) den Kern (2) vollständig umschließt, dadurch gekennzeichnet, dass die Ummantelung (3) mit einem Wirkstoff (4) versetzt ist.

## Beschreibung

Die vorliegende Erfindung betrifft Nanoteilchen zur Verabreichung eines Wirkstoffs und ein Verfahren zur Herstellung von Nanoteilchen zur Verabreichung eines Wirkstoffs. Weiters betrifft die Erfindung Nanoteilchen zur Verwendung als Arzneimittel und ein Arzneimittel, umfassend ein Nanoteilchen.

### HINTERGRUND DER ERFINDUNG

Die medikamentöse Behandlung bildet zusammen mit der chirurgischen Operation und der Strahlentherapie die wichtigsten Ansätze in der Tumortherapie. Lange Zeit war die systemische Verabreichung von zellwachstumshemmenden Wirkstoffen die einzige Wahl in der medikamentösen Tumortherapie. Die Wirkstoffe werden durch die intravenöse Verabreichung über die Blutbahn im ganzen Körper verteilt. Anstelle dieser konventionellen systemischen Behandlungsmethode können Wirkstoffe auch zielgerichtet an Tumorzellen adressiert werden.

Zur zielgerichteten Verabreichung von Wirkstoffen werden inzwischen auch magnetisierbare Nanoteilchen als Wirkstoffabgabesystem eingesetzt. Zum Beispiel offenbart US 9,393,396 B2 ein Verfahren und eine Zusammensetzung von beschichteten Nanoteilchen zur lokalen hyperthermischen Behandlung von Tumorzellen. Die mit einem Wirkstoff angereicherten Nanoteilchen werden mit Hilfe eines Magnetfeldes an eine definierte anatomische Stelle im Körper geleitet, wo die Nanoteilchen den Wirkstoff abgeben können.

Wan et al. beschreiben in "Stable and Biocompatible Colloidal Dispersions of Superparamagnetic Iron Oxide Nanoparticles with Minium Aggregation for Biomedical Applications", J Phys Chem C 120 (2016) 23799-23806, https://doi.org/10.1021/acs.jpcc.6b06614, superparamagnetische Eisenoxid-Nanoteilchen, die durch ein Polyolverfahren hergestellt und mit Natriumtripolyphosphat (TPP) beschichtet werden. TPP kann an der Oxidoberfläche verankert werden, indem es stabile Bindungen mit den Phosphatgruppen eingeht. Diese Nanopartikel behalten ihre Größe, Morphologie und nicht-aggregierende Beschaffenheit und können in wässriger Lösung über Jahre hinweg stabil bleiben. Diese Nanopartikel werden als Kontrastmittel verwendet.

Le-Vinh et al. zeigen in "Alkaline Phosphatase: A Reliable Endogenous Partner for Drug Delivery and Diagnostics" Adv Therap, 5(2) (2022) 2100219, https://doi.org/10.1002/adtp.202100219, dass alkalische Phosphatase (ALP) für die gezielte Verabreichung von Arzneimitteln und die Diagnostik wichtig ist. ALP wird als bedeutend für die "Aktivierung" von phosphorylierten Pro-Drugs, Phosphat-gesteuerten Drug-Delivery-Systemen und ALP-getriggerten Sonden für die Diagnostik beschrieben. Die folgenden Systeme werden als ALP (alkalische Phosphatase)-bezogene Arzneimittelabgabesysteme aufgeführt: Systeme zur Umwandlung von Oberflächenladungen, selbstorganisierende Nanofasern und Hydrogele, reversible Sol-Gele, multireaktive Hydrogele sowie (dis)aggregierende Nanoträger. Es wird auch beschrieben, dass Polyphosphate in der Biomedizin für die Verabreichung von Medikamenten und als Beschichtungsmaterialien verwendet werden können, wobei auch Tripolyphosphat (TPP) und Phytinsäure (PA) als Substrat für ALPs (alkalischen Phosphatasen) erwähnt werden.

### KURZBESCHREIBUNG DER ERFINDUNG

Die im Stand der Technik verwendeten Behandlungsmethoden zur präzisen Abgabe eines Wirkstoffs sind in Bezug auf Effizienz, (Off-Target-) Zytotoxizität und Wirksamkeit noch immer unzureichend. Dies liegt unter anderem daran, dass der Wirkstoff nicht zwischen Tumorzellen und gesunden Zellen unterscheiden kann und die Nanoteilchen nicht präzise genug im Körper positioniert werden können. Der Wirkstoff wird weder spezifisch genug an die Zielzellen adressiert, noch kann der Wirkstoff durch Abgabe an und Aufnahme durch die Zielzellen (ausreichend) metabolisiert werden. Aufgabe der vorliegenden Erfindung ist daher die Bereitstellung von Nanoteilchen zur Verabreichung eines Wirkstoffs im Körper, welche exakter positionierbar sind und den Wirkstoff zielgerichteter an die gewünschten Zielzellen abgeben, um gesunde Zelle zu schonen und die Wirksamkeit und (Off-Target-) Zytotoxizität freier Wirkstoffe zu verringern.

Gelöst wird diese Aufgabe durch Nanoteilchen zur Verabreichung eines Wirkstoffs, umfassend einen magnetisierbaren Kern, wobei der Kern eine Ummantelung aus einem Polyphosphat aufweist, wobei die Ummantelung den Kern vorzugsweise vollständig umschließt, dadurch gekennzeichnet, dass die Ummantelung mit einem Wirkstoff versetzt ist.

Der Begriff magnetisierbarer Kern bezieht sich sowohl auf einen Kern, der geeignet ist, magnetisiert zu werden als auch auf einen permanent magnetischen Kern.

Unter dem Begriff Wirkstoff ist sowohl ein Reinstoff als auch ein Wirkstoffgemisch zu verstehen.

Die Erfinder haben herausgefunden, dass eine Ummantelung des Kerns mit einem Polyphosphat die Aufnahme bzw. Bindung eines Wirkstoffs ermöglicht. Die Ummantelung aus Polyphosphat ermöglicht die Aufnahme einer definierten Wirkstoffmenge. Über ein externes Magnetfeld können die Nanopartikel im Körper präzise positioniert werden. Die Ummantelung aus Polyphosphat erlaubt aber auch die Freisetzung des Wirkstoffs an der gewünschten Stelle im Körper aufgrund der physiologischen Bedingungen im Körper.

Die Ummantelung aus Polyphosphat ist überdies vorteilhaft, da es sich bei Polyphosphat um ein vom Körper gut verträgliches biodegradierbares Polymer handelt, welches von der FDA (U.S. Food and Drug Administration) als "*Generally RecognizedAs Safe (GRAS)"* ("*allgemein als sicher anerkannt"*) eingestuft ist. Polyphosphat wird unter anderem als Nahrungsergänzungsmittel verwendet.

Für die Verträglichkeit im Organismus ist außerdem eine vollständige Ummantelung des Kerns mit der Ummantelung aus Polyphosphat vorteilhaft.

Der Wirkstoff ist an das Polyphosphat bevorzugt ionisch gebunden. Da Polyphosphate bei physiologischem pH-Wert in wässriger Umgebung negativ geladen sind bzw. als Anion vorliegt, ist es vorteilhaft, wenn der Wirkstoff positiv geladen ist bzw. als Kation vorliegt, sodass der Wirkstoff und das Polyphosphat eine ionische Bindung eingehen können. Anhand der negativen Ladungsträger des Polyphosphats und der positiven Ladungsträger des Wirkstoffs kann stöchiometrisch die Menge an Wirkstoff eingestellt werden. Auf diese Weise kann eine effiziente und definierte Aufnahme des Wirkstoffs durch das Polyphosphat erreicht werden. Außerdem kommt es zu einer Verringerung der (Off-Target-) Zytotoxizität. Das Massenwirkungsgesetz führt dazu, dass Polyphosphatgebundener Wirkstoff an die physiologische Umgebung (Körperflüssigkeit) abgegeben wird. Da der Wirkstoff im Körper ständig abtransportiert wird, erfolgt eine kontinuierliche Freisetzung des Wirkstoffs. Die Nanopartikel ermöglichen daher eine sustained-release Dosierung, d. h. eine modifizierte oder anhaltende Freisetzung des Wirkstoffs.

Die Menge an Wirkstoff, der von der Ummantelung aufgenommen werden kann, kann durch die Menge an vorhandenem Polyphosphat reguliert werden kann. Dies kann beispielsweise - wie oben ausgeführt - durch die negativen Ladungsträger am Polyphosphat und die positiven Ladungsträger am Wirkstoff erfolgen. Auf diese Weise kann die Dosierung bzw. die Dosierungsgenauigkeit des Wirkstoffs begünstigt und gleichzeitig die Zytotoxizität reduziert werden.

Erfindungsgemäß sind demzufolge Wirkstoffe bevorzugt, welche zumindest eine kationische Ladung aufweisen. So ist es möglich, dass der kationisch geladene Wirkstoff von den von der Ummantelung gebildeten Anionen - nämlich Phosphaten - ionisch gebunden wird. Der kationisch geladene Wirkstoff ist bevorzugt ausgewählt aus der Gruppe bestehend aus Zytostatikum, Hormon, Antikörper, Fusionsprotein, Peptide, Zytokin, Signaltransduktions-Inhibitor, Protease-Inhibitor, Proteasom-Inhibitor, HMG-CoA-Reduktase-Inhibitor, Analgetikum und Mischungen daraus. Die genannten Wirkstoffe sind vorteilhaft, da sie ein breites Spektrum an Zielmolekülen (Targets) im Körper abdecken. Beispielsweise kann ein Signaltransduktions-Inhibitor ein Angiogenese-Inhibitor sein, welcher die Regression pathologischer stromaler Blutgefäße im Tumor fördert.

Die Anwendung des Systems ist nicht auf Humanbehandlung beschränkt, sondern auch in der Veterinärmedizin möglich.

In einer Ausführungsform liegt der Wirkstoff als physiologisch annehmbares Salz vor, wobei der Wirkstoff kationisch ist.

Der Kern ist vorzugsweise superparamagnetisch oder ferritisch. Superparamagnetische Kerne weisen gegenüber ferritischen Kernen den zusätzlichen Vorteil auf, dass sie keine agglomerierten Aggregate bilden und vereinzelt vorliegend bleiben, sodass superparamagnetische Kerne bevorzugt sind. Diese werden in der internationalen Fachliteratur auch unter dem Begriff SPIONs (superparamagnetic iron oxide nanoparticles) zusammengefasst.

In einer Ausführungsvariante ist vorgesehen, dass der Kern Fe₂O₃ bzw. Fe₃O₄ umfasst, vorzugsweise daraus besteht. In einer weiteren Ausführungsvariante ist vorgesehen, dass der Kern Maghemit (γ-Fe₂O₃), Cobaltferrit (CoFe₂O₄) oder Mischungen daraus umfasst bzw. daraus besteht.

Die Ummantelung aus Polyphosphat umfasst bevorzugt Tripolyphosphat (TPP), Natriumpolyphosphat (PP), Pentapolyphosphat, Hexametaphosphat, Graham's Salz, Phytinsäure (PA), Guanosintriphosphat (GTP), Adenosintriphosphat (ATP) und/oder Mischungen daraus. Diese Polyphosphate eignen sich zur Bildung stabiler Bindungen mit oxidischen Bestandteilen des Kerns. Daher sind diese Polyphosphate insbesondere vorteilhaft, wenn der Kern Fe₂O₃ bzw. Fe₃O₄ umfasst, vorzugsweise daraus besteht. Der Kern wird dadurch auch unzugänglich für Körperflüssigkeiten. Der genaue Bindungsmechanismus zwischen dem oxidischen Bestandteil des Kerns und dem Polyphosphat ist unklar, er dürfte aber eine Kombination aus ionischer Bindung und Wasserstoffbrückenbindungen ggf. auch kovalente Bindungen umfassen, sodass die Bindungsstärke ähnlich oder sogar stärker als die Bindung zwischen der negativen Ladung des Phosphats und der positiven Ladung eines Wirkstoffs ist. Dadurch haftet das Polyphosphat am Kern, auch wenn der Wirkstoff bereits abgegeben wurde.

In einer weiteren Ausführungsform kann die Ummantelung aus Polyphosphat zusätzlich eine Verbindung aus der Gruppe bestehend aus Dextran, Carboxydextran, Carboxymethyldextran, PEG-starch, Siloxane, sulfonierte Styrenedivinylbenzene, Copolymer und Mischungen daraus umfassen.

Die in den beiden vorherigen Absätzen genannten Ausführungsformen sind besonders vorteilhaft, da die Nanoteilchen ihre Größe, Morphologie und nicht-aggregierende Natur beibehalten. Zudem verfügen sie über eine jahrelange Lagerstabilität in wässriger Lösung als auch in lyophilisierter Form.

Aus dem Zusammenspiel der Komponenten Kern/Ummantelung/Wirkstoff kann festgelegt werden, wie lange der Wirkstoff im bzw. am Nanoteilchen gehalten werden kann. In einer Ausführungsform kann die Kombination dieser drei Komponenten so gewählt sein, dass beispielsweise der positiv geladene Wirkstoff an die negativ geladene Phosphatgruppe bindet. Gleichzeitig kann die Bindung des Wirkstoffs an die Phosphatgruppe so sein, sodass der Wirkstoff bei physiologischen Bedingungen an den Körper abgegeben und von den Zielzellen metabolisiert werden kann und dass der Wirkstoff nicht am Nanoteilchen verbleibt. Während der Wirkstoff abgegeben wird, kann das Polyphosphat am Kern haften bleiben. Eine ausgewogene Bindungsstärke zwischen Wirkstoff und Ummantelung kann somit vorteilhaft sein. Wenngleich Polyphosphate sehr stark an Eisenoxide gebunden werden [Wan et al., Molecular Mechanism of Linear Polyphosphate Adsorption on Iron and Aluminum Oxide. J. Phys. Chem. C 2020, 124, 52, 28448-28457], konnte im Rahmen dieser Erfindung erstmals gezeigt werden, dass diese Polyphosphate dennoch durch ALP enzymatisch abgespalten werden können.

Auch ist es möglich, die Masse der Ummantelung von der zu verabreichenden Wirkstoffmenge und dem Wirkstoff abhängig zu gestalten, sodass in einer Ausführungsform die Schichtdicke nicht weniger als 5 %, vorzugsweise zumindest 20 %, bezogen auf die Gesamtmasse des Nanoteilchens betragen sollte.

Beispielhaft kann der Kern mit Ummantelung einen Durchmesser von 50 nm bis 850 nm, vorzugsweise 100 nm bis 800 nm, besonders bevorzugt 150 nm bis 750 nm aufweisen. Für den menschlichen Organismus hat sich diese Partikelgröße als vorteilhaft herausgestellt, während in der Veterinärmedizin auch kleinere Größen verwendet werden können, wie beispielsweise im Bereich von 10 bis 30 nm.

Speziell für die humanmedizinische Anwendung ist ein Durchmesser des Kerns mit Ummantelung von nicht unter 50 nm vorteilhaft, da Partikel mit Durchmesser unter 50 nm vom menschlichen Körper oft mit großer Verzögerung ausgeschieden werden können. Teilchen kleiner als 10 nm haben sich überhaupt als zellgängig erwiesen und könnten daher in Zellen eindringen, was im Rahmen der Erfindung vermieden werden soll.

Die Erfindung betrifft außerdem Nanoteilchen der vorgenannten Art zur Verwendung als Arzneimittel beispielsweise in der Humanmedizin oder Veterinärmedizin, insbesondere in der Onkologie, Immunologie, Hämatologie und/oder Schmerztherapie. In der onkologischen und immunologischen Anwendung sind die oben genannten Krankheitsbilder bevorzugt.

Ein bevorzugtes Anwendungsgebiet in der Onkologie ist die Behandlung von soliden Tumoren. Dazu zählen beispielsweise Mammakarzinom, Lungenkarzinom, Kolonkarzinom, Rektalkarzinom, Blasenkarzinom, hepatozelluläres Karzinom, Ovarialkarzinom, Prostatakarzinom, Melanom, Sarkom (Osteosarkom, Lipsarkom, Leiomyosarkom), Lymphom, neuroendokriner Tumor, Keimzellentumor, Gliom (Astrozytom, Glioblastom, Oligodendrogliom, Ependymom) Blastom etc. Auch wenn solide Tumore in der Regel in einem Organ lokalisiert sind, ist auch die Behandlung deren Metastasen in entfernten Körperregionen möglich. Zudem ist nicht nur die Behandlung von malignen Tumoren möglich sondern auch die Behandlung von benignen Tumoren, wie beispielsweise Myome, Lipome, Adenome, Fibrome, Angiome, Chondrome, fibröse Dysplasien, Riesenzelltumore etc. Daher ist beim Nanoteilchen zur Verwendung als Arzneimittel der Wirkstoff bevorzugt ein Zytostatikum.

In einem weiteren Aspekt der Erfindung liegen die Nanoteilchen mit Wirkstoff in getrockneter Form vor. Daher kann die Erfindung auch ein Kit umfassen, welches die Nanoteilchen der vorgenannten Art in einem Behälter und einen physiologisch annehmbaren Puffer in einem getrennten Behälter sowie eine Spritze umfasst, wobei das Kit für Verwendung in der Zubereitung einer gebrauchsfertigen Suspension aus Nanoteilchen im Puffer unmittelbar vor der Injektion der Suspension dient. Der physiologisch annehmbare Puffer weist bevorzugt einen pH-Wert in einem Bereich von etwa 3,0 bis etwa 10,0, bevorzugt von etwa 3,5 bis etwa 8,0 auf. In diesem Bereich zeigt sich eine hohe Lagerstabilität bei verschiedenen Lagertemperaturen. In einer Ausführungsform stimmt der pH-Wert des Puffers mit dem physiologischen Gewebe- und Blut-pH-Wert von 7,35 bis 7,55 überein.

In einer weiteren Ausführungsform ist der Puffer ein Phosphat-Puffer, ein Tris-Puffer, ein Zitrat-Puffer, ein Acetat-Puffer, bevorzugt ein Natriumacetat-Puffer, und liegt beispielsweise in einer Konzentration zwischen etwa 10 mM und etwa 1000 mM vor.

In einer Ausführungsform liegen die Nanoteilchen und/oder der Wirkstoff des Kits in lyophilisierter Form vor. Zur Herstellung einer gebrauchsfertigen Infusionslösung kann das Lyophilisat durch Zugabe einer erforderlichen Menge eines Puffers wie beispielsweise 0,9 %-iger NaCl-Lösung oder 5 %-iger Glukoselösung rekonstituiert bzw. verdünnt werden. In einer Ausführungsform kann dies direkt in einem Fläschchen als Behälter, d. h. in einem Durchstechfläschchen stattfinden. Dies kann vorteilhaft sein, da das Lyophilisat einfacher und länger haltbar ist und sich durch eine hohe Lagerstabilität auszeichnet.

Die oben genannten Ausführungsformen schließen sich nicht gegenseitig aus. So können beispielsweise in einer weiteren bevorzugten Ausführungsform die Nanoteilchen in einem Puffer mit physiologisch annehmbaren pH-Wert vorliegen und der Wirkstoff als Lyophilisat verfügbar sein, oder umgekehrt.

Die Erfindung betrifft auch eine vorgefüllte Spritze, umfassend
- einen Puffer mit einem physiologisch annehmbarem pH-Wert,
- Nanoteilchen der oben genannten Art mit einem Wirkstoff.

In einer Ausführungsform stellt die Erfindung eine Verpackung, wie z. B. eine Kartonverpackung bereit, die eine erfindungsgemäße vorgefüllte Spritze in einer Blisterpackung, eine Nadel und gegebenenfalls eine Gebrauchsanweisung enthält.

Die Erfindung betrifft außerdem eine Spritze, umfassend
- einen Puffer mit einem physiologisch annehmbarem pH-Wert,
- Nanoteilchen der oben genannten Art,
- einen Wirkstoff der oben genannten Art, wobei der Wirkstoff in dem Puffer mit physiologisch annehmbaren pH-Wert positiv geladen ist.

In einer Ausführungsform enthält die Spritze ein Nanoteilchen ohne Ummantelung aus Polyphosphat, das bedeutet, dass das Nanoteilchen in nicht ummantelter Form vorliegt. Die Ummantelung aus Polyphosphat kann in einem zusätzlichen Arbeitsschritt, kurz vor Applikation des erfindungsgemäßen Gegenstands, hinzugefügt werden.

In einer weiteren Ausführungsform enthält die Spritze bereits ein Nanoteilchen mit Ummantelung aus Polyphosphat.

In einer Ausführungsform wird der erfindungsgemäße Gegenstand bevorzugt lokal appliziert. Dabei können die Nanoteilchen entweder über einen Katheter in das Zielorgan bzw. Zielgewebe eingeschwemmt werden oder aber mittels einer Spritze mit geeigneter Kanüle intradermal, intrakutan, subkutan, intramuskulär, intravitreal, intralymphatisch, intraartikulär, intralumbal, intraperitoneal, ventrogluteal injiziert werden. Gegebenenfalls kann ein Trokar zu Hilfe genommen werden.

In einer weiteren Ausführungsform können die erfindungsgemäßen Nanoteilchen systemisch wie beispielsweise intravenös appliziert werden. Alternativ ist eine intravenöse Verabreichung über ein Portsystem möglich. Das Portsystem ist vor allem bei onkologischen Patienten bevorzugt, die über schlechte Venenverhältnisse verfügen und über mehrere Zyklen hinweg wiederholt behandelt werden. In einer weiteren Ausführungsform kann der erfindungsgemäße Gegenstand auch über einen ZVK (zentralvenösen Katheter) appliziert werden. Vor allem für längerfristige Anwendungen bzw. die Anwendung gefäßtoxischer hyperosmolarer Substanzen und Zytostatika kann ein Katheter in einer größeren, zentralen Vene vorteilhaft sein.

In einem Aspekt der Erfindung ist auch ein Verfahren zur Positionierung von Nanoteilchen der oben genannten Art vorgesehen, welches ein magnetisches Objekt, vorzugsweise ein stabförmiges magnetisches Objekt, an der Außenseite eines Körpers an der Epidermis positioniert wird.

In einem Aspekt der Erfindung ist auch ein Verfahren zur Positionierung von Nanoteilchen der oben genannten Art vorgesehen, welches ein magnetisches Objekt, vorzugsweise ein stabförmiges magnetisches Objekt, an der Außenfläche von zu therapierenden Körperbereichen bzw. an der Grenzfläche zwischen gesunden und zu therapierenden Körperbereichen (im Rahmen eines chirurgischen Eingriffs) temporär im Körper positioniert wird.

Die Erfindung betrifft auch ein Verfahren zur Herstellung eines Nanoteilchen zur Verabreichung eines Wirkstoffs der oben genannten Art. Das Verfahren umfasst die Schritte:
(a) Bereitstellen eines magnetisierbaren Kerns, wobei der Kern ein Eisenoxid umfasst,
(b) Bereitstellen einer Mischung aus Polyphosphat mit einem Wirkstoff und
(c) Ummanteln des magnetisierbaren Kerns mit der Mischung aus Polyphosphat mit Wirkstoff.

Bevorzugt werden die nach Schritt (c) erhaltenen Nanoteilchen in einem Schritt (d) getrocknet.

Als Kerne kommen Teilchen zum Einsatz, welche magnetisierbar bzw. magnetisch sind und wie oben definiert aufgebaut sein können. Abhängig von der gewünschten Dicke aus Kern und Ummantelung erfolgt auch die Auswahl der gewünschten Masse der Ummantelung bezogen auf die Gesamtmasse des Nanoteilchens. Die Gesamtmasse des Nanoteilchens umfasst den Kern und die Ummantelung. So kann die Ummantelung vorzugsweise so gewählt sein, dass sie zumindest 5 %, vorzugsweise 20 % bezogen auf die Gesamtmasse des Nanoteilchens beträgt.

Der Wirkstoff mit Polyphosphat wird bevorzugt als wässrige Lösung aufgebracht. Dabei ist es möglich, dass der Wirkstoff gemeinsam mit der Ummantelung aufgebracht wird, oder es kann der Wirkstoff nachträglich in die Ummantelung eingebracht werden (z. B. mittels Sprühtechnik eines gelösten oder suspendierten Wirkstoffs und anschließender Trocknung des Lösungsmittels oder Suspensionsmittels).

Entsprechend betrifft die vorliegende Erfindung ein Nanoteilchen wie oben definiert oder ein Arzneimittel, umfassend das Nanoteilchen, zur (Verwendung bei der) Behandlung einer Krankheit wie oben definiert, die Verwendung des Nanoteilchen oder des Arzneimittel bei der Behandlung einer Krankheit wie oben definiert und/oder die Verwendung des Nanoteilchen bei der Herstellung eines Arzneimittels zur Behandlung einer Krankheit wie oben definiert.

In einem weiteren Aspekt bezieht sich die vorliegende Erfindung auf ein Verfahren zur Behandlung einer Krankheit wie oben definiert, umfassend die Verabreichung eines Nanoteilchens wie oben definiert an ein Subjekt, das dies benötigt.

### DETAILLIERTE BESCHREIBUNG DER ERFINDUNG

Bei der gegenständlichen Erfindung handelt es sich um Nanopartikel zur spezifischen Verwendung in der Therapie, insbesondere in minimal-invasiver, lokalisierter, personalisierbarer und zielgerichteter Tumortherapie oder Behandlung mittels geeigneter Wirkstoffe bei gleichzeitig maximaler Schonung von gesunden Zellen bzw. daran angrenzender gesunder Gewebsbereiche sowie minimaler (Off-Target-) Zytotoxizität und minimalen Nebenwirkungen.

Weitere Details und Vorteile der Erfindung sind nachfolgend sowie anhand der beiliegenden Figuren und der nachfolgenden Figurenbeschreibung dargelegt.
- Fig. 1: zeigt eine schematische Darstellung zur Synthese erfindungsgemäßer Nanoteilchen.
- Fig. 2: zeigt eine Grafik zur Charakterisierung von ummantelten Nanoteilchen.
- Fig. 3A: zeigt eine Grafik zur enzymatischen Phosphatabspaltung von ummantelten Nanoteilchen.
- Fig. 3B: zeigt eine Grafik zur Zellviabilität.
- Fig. 3C: zeigt eine Grafik zur Versetzungs-Effizienz (encapsulation efficiency) von Ethacridin-Lactat (ETH) beladenen ummantelten Nanoteilchen.
- Fig. 3D: zeigt eine Grafik zur Charakterisierung von Ethacridin-Lactat (ETH) beladenen ummantelten Nanoteilchen hinsichtlich Partikelgröße, Polydispersitätsindex (PDI) und Zetapotential.
- Fig. 4A: zeigt eine schematische Darstellung der angewandten Methoden zur Wirkstofffreisetzung mittels Dialysemembran.
- Fig. 4B: zeigt eine schematische Darstellung der angewandten Methode zur Wirkstofffreisetzung mittels Zentrifugation.
- Fig. 5: zeigt eine Grafik zur Wirkstofffreisetzung von mit Wirkstoff beladenen Nanoteilchen.
- Fig. 6A: zeigt eine schematische Darstellung eines erfindungsgemäßen Systems.
- Fig. 6B: zeigt die schematische Anwendung des Systems bei der Behandlung im Körper.

Die gegenständliche Behandlungsmethode beruht im Wesentlichen auf zwei Komponenten:

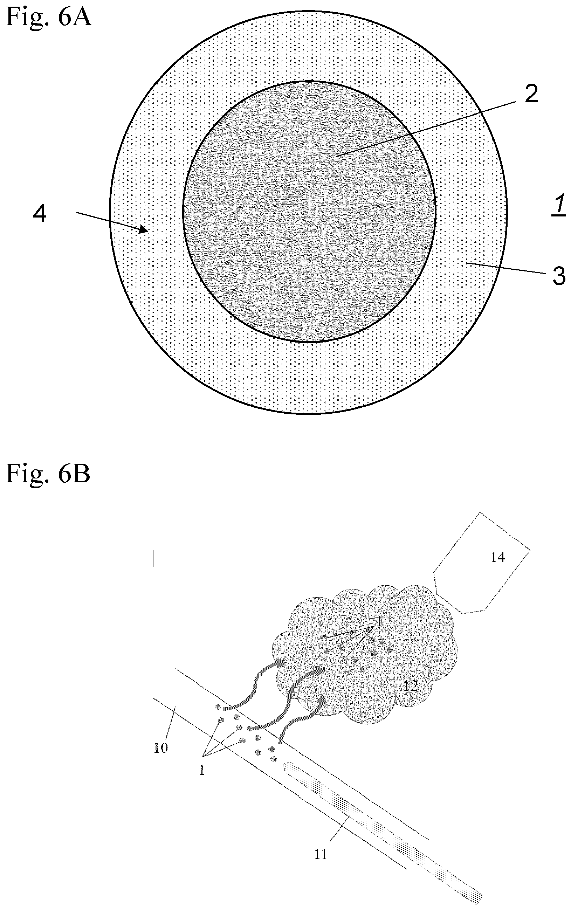

Die erste Komponente (Fig. 6A) sind biokompatible Nanoteilchen 1 mit einem magnetisierbaren Kern 2, insbesondere mit einem superparamagnetischen oder ferritischen Kern, beispielsweise Fe₂O₃ bzw. Fe₃O₄ oder Maghemit (γ-Fe₂O₃) oder Cobaltferrit (CoFe₂O₄), die ummantelt sind mit einem Polyphospha 3. Die Ummantelung aus Polyphosphat 3 kann aus einem linearen, verzweigten und/oder zyklischen Polyphosphat sowie aus Polyphosphat-Derivaten aufgebaut sein.

Die Polyphosphat-Ummantelung verleiht einen anionischen Charakter, der eine ionische Bindung eines kationischen Wirkstoffes ermöglicht. Besonders geeignete Polyphosphate sind Tripolyphosphat (TPP), Natriumpolyphosphat (PP), Pentapolyphosphat, Hexametaphosphat, Graham's Salz, Phytinsäure (PA), Guanosintriphosphat (GTP), Adenosintriphosphat (ATP) und/oder Mischungen daraus.

Die Ummantelung aus Polyphosphat ist mit dem zumindest einen Wirkstoff versetzt. Der Wirkstoff, vorzugsweise kationisch, ist an das (anionische) Polyphosphat gebunden. Polyphosphate können auch als freie Säuren vorliegen und daher keine Gegen-Ionen aufweisen, die sie zum Salz machen. Für den erfindungsgemäßen Gegenstand können in einer Ausführungsform sowohl Salze als auch freie Säuren vorteilhaft sein, Salze sind allerdings bevorzugt, da sie den Wirkstoff kontrollierter freigeben.

Die zweite Komponente (Fig. 6B) umfasst einen Magneten 14, beispielsweise durch ein magnetisierbares bzw. magnetisches Objekt, vorzugsweise einen stabförmigen Permanent- oder Elektromagneten, repräsentiert. Über das Magnetfeld des Magneten lassen sich die Nanoteilchen an den gewünschten Ort eines zu behandelnden Körpers leiten, um die Nanoteilchen mit Wirkstoff zu Zielzellen zu lenken, wo eine gezielte Wirkstoffabgabe erfolgt.

Die charakteristischen Abmessungen dieser Komponente hängen im Wesentlichen von der (räumlichen) Ausdehnung bzw. Verteilung der zu behandelnden Zielzellen bzw. der zu behandelnden Körperregion ab. Die Geometrie der zweiten Komponente hängt von der gewählten Applikationstechnik ab.

Während die Nanoteilchen lokal oder systemisch injiziert werden, erfolgt die Platzierung des Magneten z. B. durch Einführung über ein Punktionsinstrument wie beispielsweise über einen Trokar in der Nähe des gewünschten Ortes, an welchem das Nanoteilchen positioniert werden soll. Die Positionierung der Nanoteilchen kann aber auch mit einem magnetisierbaren bzw. magnetischen Objekt in Form eines Stabes erfolgen, welcher ein Magnetfeld aufweist, wobei der Stab an der Außenseite eines Körpers, an der Epidermis, an der gewünschten Stelle positioniert wird.

Die Erfindung bietet sich vor allem für eine neuartige Tumortherapie an und sieht vorzugsweise die zeitgleiche und sehr präzise Applikation der beiden Komponenten im Nahebereich des zu behandelnden Gewebes (Nanoteilchen werden intrakorporal, Magnet wird intrakorporal oder an der Körperoberfläche, d. h. extrakorporal, positioniert) ausgehend von zwei unterschiedlichen Positionen vor. Die Zeitspanne zwischen der Applikation der beiden Komponenten kann auch eine definierte Dauer darstellen, um die Wirkstofffreisetzung bzw. die Wirkung des Wirkstoffs auf die Zielzellen bzw. die Körperregion zu optimieren.

Die mit Wirkstoff beladenen Nanoteilchen werden über die oben genannten Injektionsmöglichkeiten direkt in das zu behandelnde Gewebe, z. B. einen Tumor und/oder in den Nahbereich des zu behandelnden Gewebes, z. B. des Tumors eingebracht und geben über einen definierten Zeitraum den Wirkstoff dosiert an die Zielzellen bzw. Zielregion im Gewebe ab. Der Magnet wird im Nahebereich der zu behandelnden Körperregion beispielsweise mittels (magnetischer) Biopsie-Nadel, Katheter und/oder Trokar positioniert. Die Nanoteilchen bleiben für eine definierte Zeitspanne (von zumindest mehreren Sekunden) im Körper bzw. im Nahebereich des zu behandelnden Gewebes und können wieder minimal-invasiv entfernt werden.

Aufgrund der magnetischen Wirkung zwischen Magnet und Nanoteilchen verbleiben die Nanoteilchen im Nahbereich der Zielzellen bzw. des Zielorgans und können dort maximale Wirksamkeit erzielen. Begünstigt wird der Wirkstoffeintrag durch eine Bewegung der Nanoteilchen, diese Bewegung kann von außen über die Biopsie-Nadel, den Trokar, ein Katheter oder den Magneten an der Körperoberfläche, z. B. mittels Oszillation, erzwungen werden.

Die Nanoteilchen verteilen sich nicht über einen weiten Bereich des Körpers und gewährleisten so eine maximale Schonung gesunder Körperregionen. Aufgrund der stark lokalisierten Wirkung dieser Tumorbehandlung kann sie vereinfachend als "Tumortherapie ohne Off-Target-Zytotoxizität und nahezu nebenwirkungsfrei" verstanden werden.

Nach Ende der Behandlungszeit können die Nanoteilchen wieder über ein magnetisches Hilfsmittel (z. B. Katheter mit Magnetspitze) gesammelt und schonend aus dem Körperinneren rückstandslos bzw. nahezu rückstandslos entfernt werden. Die nicht mit einem magnetischen Hilfsmittel entfernten Kerne mit Ummantelung werden nach einer gewissen Zeit automatisch ausgeschieden. Da Nanoteilchen leicht verstoffwechselt und aus dem Organismus extrahiert werden können, kann die Ausscheidung beispielsweise über die Niere oder über die Galle in den Darm und weiter über den Stuhl erfolgen. Die im Blut zirkulierenden Nanoteilchen können durch das mononukleär-phagozytäre System beispielsweise über Phagozytose, Makropinozytose, Opsonisierung und/oder Caveolin-vermittelte Endozytose aufgenommen und über verschiedene Organe wie Leber und/oder die Milz ausgeschieden werden.

### Synthese und Charakterisierung von Nanoteilchen (nicht ummantelt und unbeladen)

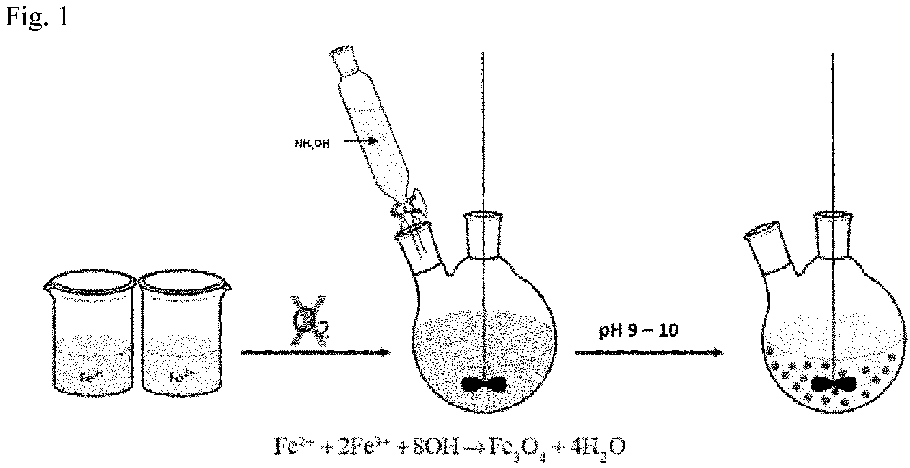

Superparamagnetische Eisenoxid-Nanoteilchen wurden durch Co-Präzipitation hergestellt (siehe Fig. 1). In einem beispielhaften Experiment wurde zunächst eine Lösung aus Eisen(II)- und Eisen(III)-Ionen in einem Molverhältnis von 1:2 hergestellt, indem 8 mmol FeCl₂•4H₂O und 16 mmol FeCl₃•6H₂O in 150 mL demineralisiertem Wasser unter Stickstoffbedingungen aufgelöst wurden. Anschließend wurde Eisenoxid durch tropfenweise Zugabe von 15 mL NH₄OH-Lösung (10 %, w/w) unter ständigem Schütteln bei 650 rpm ausgefällt, bis der pH-Wert einen Bereich zwischen 9 und 10 erreichte und die Lösung sich dunkel schwarz färbte. Die Mischung wurde 1 Stunde lang bei Raumtemperatur gerührt. Anschließend wurde der Niederschlag mit einem Magnetstab aus der Lösung isoliert und mindestens dreimal mit entionisiertem Wasser gewaschen, bis ein pH-Wert von 7 erreicht war. Die gereinigten Nanoteilchen wurden schließlich in 150 mL entmineralisiertem Wasser resuspendiert und zur weiteren Verwendung im Kühlschrank aufbewahrt.

Um die Stabilität der nicht ummantelten Nanoteilchen zu überprüfen, wurde eine Charakterisierung hinsichtlich Partikelgröße, Polydispersitätsindex (PDI) und Zetapotenzial durchgeführt (siehe Tabelle 1).

**Tabelle 1. Charakterisierung von nicht ummantelten Nanoteilchen (n=3 ±SD).**

| **Dispersionsmedium** | **Partikelgröße [nm]** | **PDI** | **Zetapotential [mV]** |
|---|---|---|---|
| **Demineralisiertes Wasser** | 2513,94 ± 97,43 | 0,29 ± 0,14 | 9,17 ± 1,58 |
| **20 mM HEPES Puffer** | 2414,13 ± 185,69 | 0,19 ± 0,06 | -12,36 ± 0,93 |

Die nicht ummantelten Nanoteilchen neigten zu Aggregatbildung in wässriger Umgebung, sodass die Partikelgröße im mm-Bereich lag. Mit der Ummantelung der Nanoteilchen nahm die Partikelgröße um zumindest einen Faktor 10 ab, sodass die Partikelgröße in den Nanobereich reduziert wurde.

### Synthese und Charakterisierung von ummantelten Nanoteilchen

Eisenoxid-Nanopartikel, die im Co-Präzipitationsverfahren hergestellt wurden, wurden mit TPP (Tripolyphosphat), PP (Natriumpolyphosphat), PA (Phytinsäure) oder ATP (Adenosintriphosphat) ummantelt. Die Ummantelungsmittel wurden in demineralisiertem Wasser in verschiedenen Konzentrationen von 15 mg/mL bis 0,25 mg/mL gelöst. Danach wurden jeder Ummantelung (*coating-agent-solution*) nicht ummantelte Nanoteilchen im Volumenverhältnis 1:10 (Nanopartikel : Polyphosphat) zugesetzt. Die so entstandenen Mischungen wurden über Nacht bei Raumtemperatur unter Schütteln bei 600 rpm inkubiert. Am darauffolgenden Tag wurden die ummantelten Nanoteilchen visuell auf ihre kolloidale Stabilität hin untersucht, mit Hilfe eines Magneten getrennt und zweimal mit demineralisiertem Wasser gewaschen. Im Rahmen von beispielhaften Orientierungsstudien wurden kleine Chargen von 1 mL ummantelter Nanoteilchen hergestellt, während der Prozess für die vielversprechendsten Träger für weitere Studien auf 50 mL aufgestockt wurde. Dabei wurden 5 mL nicht ummantelte Nanoteilchen zu 45 mL wässrigen Lösungen mit 8 mg/mL TPP, 4 mg/mL PP, 15 mg/mL PA und 1 mg/mL ATP hinzugefügt. Die resultierenden Mischungen wurden wie oben beschrieben behandelt und gereinigt.

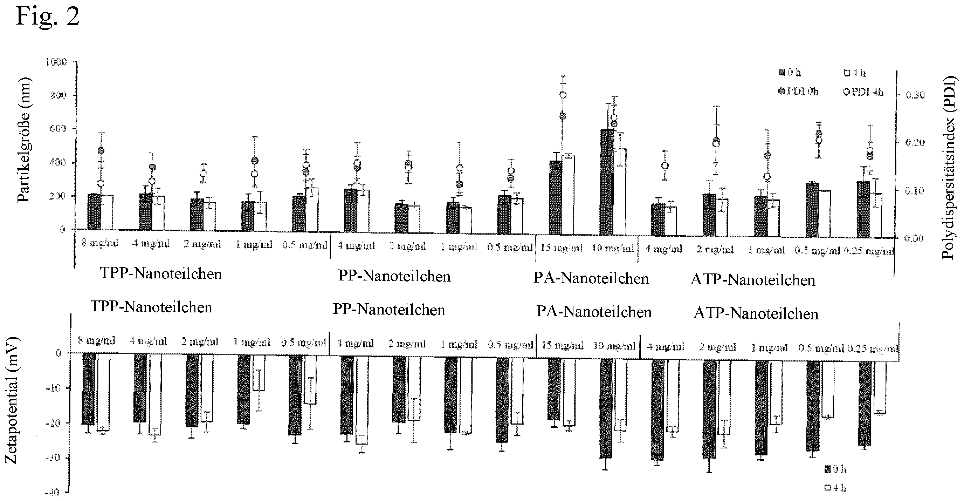

Um die Stabilität der ummantelten Nanoteilchen zu überprüfen, wurde eine Charakterisierung hinsichtlich Partikelgröße, Polydispersitätsindex (PDI) und Zetapotenzial durchgeführt (siehe Fig. 2). Dabei wurden die mit TPP, PP und PA ummantelten Nanoteilchen 1:1 (v/v) mit 20 mM HEPES-Puffer pH 7,4 verdünnt und auf 37°C vorgewärmt. Aliquote wurden sofort nach der Herstellung sowie nach einer 4-stündigen Inkubation bei 37°C unter Schütteln bei 300 rpm entnommen und nach weiterer Verdünnung in Wasser im Verhältnis 1:10 bzw. 1:5 (v/v) für TPP/PP- und PA-ummantelte Partikel analysiert. ATP-ummantelte Nanoteilchen wurden direkt nach Verdünnung im Verhältnis 1:10 (v/v) mit 20 mM HEPES-Puffer pH 7,4 unmittelbar nach der Herstellung und nach 4 Stunden Inkubation (37°C und 300 rpm) analysiert (n=4 ± SD).

Wie in Fig. 2 dargestellt, wurden die magnetischen Nanoteilchen mit verschiedenen getesteten Mengen von TPP, PP, PA oder ATP ummantelt. So konnte unter anderem die Aggregation der Teilchen verhindert werden. Einerseits zeigten Nanoteilchen, die mit linearem Polyphosphat nach der Zugabe von Ummantelungsmitteln (d. h. TPP, PP, PA oder ATP) ummantelt waren, bei allen untersuchten Konzentrationen eine geringere Tendenz zur Aggregation, was durch die kleinere Teilchengröße von etwa 200 nm bis 300 nm sowie durch das negative Zetapotenzial von etwa -20 mV unterstrichen wird (siehe Fig. 2). TPP-ummantelte Nanoteilchen, die mit einer Menge von weniger als 1 mg/mL Ummantelungsmittel (*coating agent*) hergestellt wurden, zeigten jedoch nach 4 Stunden Inkubation eine Instabilität, was durch die Auswertung des Zetapotenzials deutlich wird. Darüber hinaus wurde bei Proben, die mit TPP in Konzentrationen von weniger als 4 mg/mL ummantelt waren, nach einer Woche Lagerung bei 2°C bis 8°C eine Aggregation beobachtet. Daher wurde TPP in einer Konzentration von 8 mg/mL für weitere Untersuchungen ausgewählt, um stabilere ummantelte Nanoteilchen zu erhalten. Bei den mit PP-ummantelten Nanoteilchen wurde die Ummantelungsmittelkonzentration von 4 mg/mL, die eine ausreichende Stabilität aufweist, für weitere Untersuchungen ausgewählt. Die PA-Konzentration von 15 mg/mL wurde für die weiteren Untersuchungen gewählt, da sich eine kleinere Partikelgröße und eine höhere Stabilität des kolloidalen Systems im Vergleich zu den mit 10 mg/mL PA ummantelten Nanoteilchen ergab. Darüber hinaus zeigten die ATP-Nanoteilchen bei der Herstellung mit ATP-Konzentrationen von weniger als 1 mg/mL eine ausreichende Stabilität, während bei höheren Konzentrationen visuell eine Ausfällung beobachtet wurde. Nach der Reinigung wiesen jedoch alle untersuchten Proben eine Partikelgröße von 200 nm bis 350 nm auf, die über 4 Stunden stabil war. Um eine mögliche Instabilität zu vermeiden, wurden ATP-ummantelte Nanoteilchen mit 1 mg/mL für die Herstellung größerer Chargen ausgewählt. Alle ummantelten Nanoteilchen wiesen einen PDI von weniger als 0,300 auf, der über 4 Stunden Inkubation stabil war.

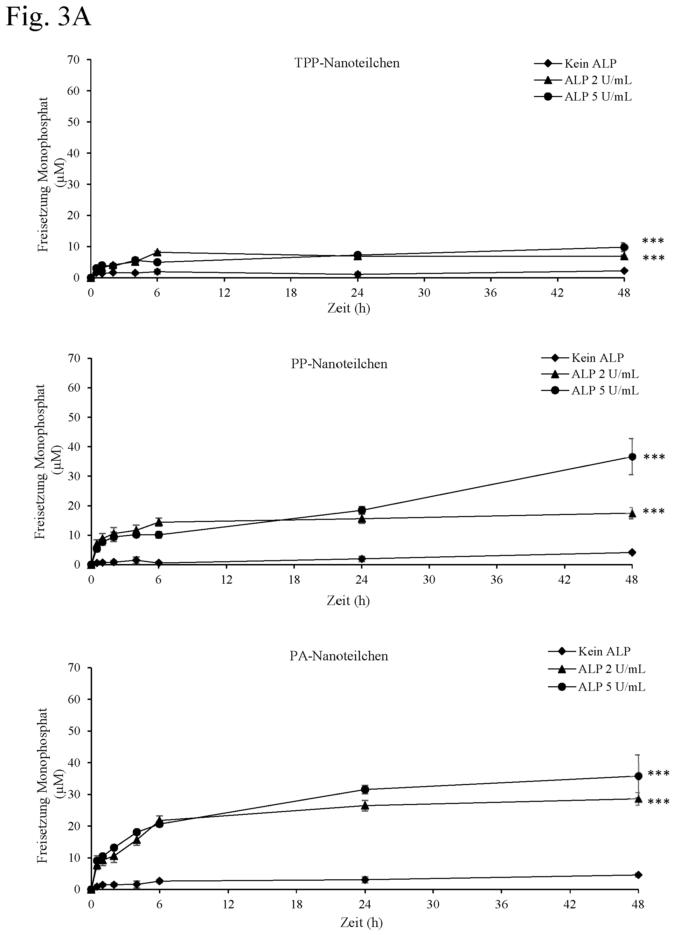

Die Enzym-induzierte Phosphatabspaltung von ummantelten Nanoteilchen wurde mittels MLG-Assay (Malachitgrün-Assay) bestimmt (siehe Fig. 3A). Die Enzym-induzierte Phosphatabspaltung wurde durch alkalische Phosphatasen (ALP) induziert, wobei ummantelte Nanoteilchen 1:10 (v/v) in 20 mM HEPES-Puffer verdünnt und über 48 h mit ALP 2 U/mL und ohne Enzym inkubiert wurden. Angegebene Werte sind Mittelwerte von n=4± SD. Signifikante Unterschiede im Vergleich zu den Kontrollproben ohne ALP sind als ***p<0,001 dargestellt.

Fig. 3A zeigt, dass die Freisetzung von Monophosphaten bei der Inkubation mit ALP zeitabhängig zunahm. Im Gegensatz dazu zeigten die Proben ohne ALP-Zusatz eine geringe Monophosphatfreisetzung, was die durch das Enzym ausgelöste Polyphosphat-Hydrolyse der ummantelten Nanoteilchen bestätigt. Die Spaltung der Ummantelungen ging in den ersten sechs Stunden schneller vonstatten, gefolgt von einer Plateauphase, die höchstwahrscheinlich auf die Sättigung des Enzyms zurückzuführen ist. Insgesamt wurde nach 48 Stunden eine höhere Freisetzung von Monophosphaten für ATP-ummantelte Nanoteilchen > PA-ummantelte Nanoteilchen > PP-ummantelte Nanoteilchen > TPP-ummantelte Nanoteilchen beobachtet. Ähnliche Werte der freigesetzten Monophosphate wurden für alle untersuchten ummantelten Träger bei Anwendung von 2 U/mL und 5 U/mL ALP (alkalische Phosphatase) festgestellt. Nur bei PP-Nanoteilchen wurde nach 48 Stunden Inkubation mit höherer Enzymkonzentration eine höhere Freisetzung beobachtet. Ein Verhalten, das mit leichter Tendenz auch bei ATP zu verzeichnen ist.

### Messungen zur Zellviabilität nach Inkubation mit ummantelten Nanoteilchen

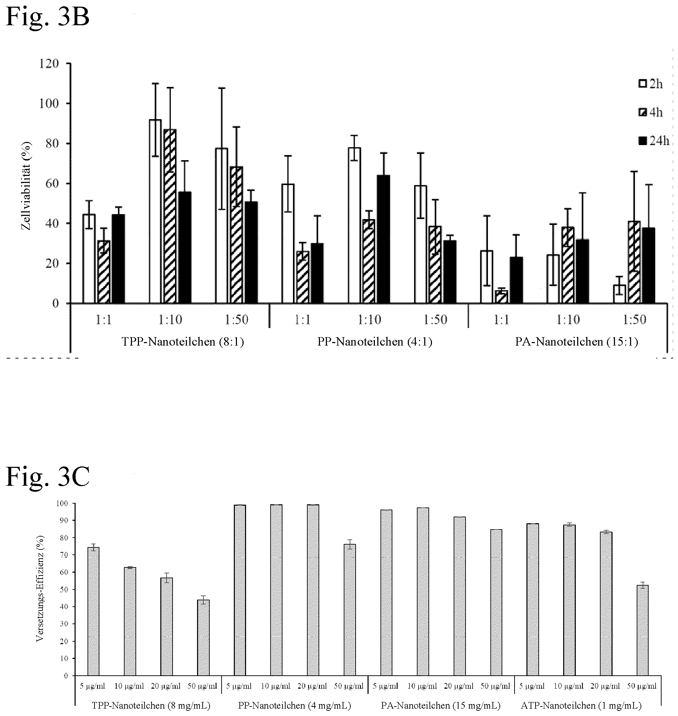

Zum Zweck einer ersten Zellviabilitätsstudie wurden, wie in Fig. 3B dargestellt, die erfindungsgemäßen Nanoteilchen mit TPP 8:1, PP 4:1 oder PA 15:1 ummantelt, wobei die Ummantelung für diese Studie nicht mit einem Wirkstoff versetzt wurde. Die Zellviabilität wurde mittels des MTT-Assay ermittelt. Der MTT-Assay ist ein kolorimetrischer Test zur Bewertung der Viabilität von Zellen, bei dem die Stoffwechselaktivität gemessen wird. Viable Zellen, die NADPH-abhängige zelluläre Oxidoreduktase-Enzyme enthalten, sind in der Lage, den Tetrazolium-Farbstoff (MTT, gelbliche Farbe) zu seinem unlöslichen Formazan (lila Farbe) zu reduzieren. Zur Messung der Zellviabilität in Zusammenhang mit dem erfindungsgemäßen Gegenstand (Nanoteilchen 1) wurde eine immortalisierte Zelllinie, nämlich humane embryonale Nierenzellen ("*human embryonic kidney cells* ") (HEK 293) verwendet. Die Kultivierung von HEK 293 Zellen ist dem Stand der Technik bekannt. Kurz gesagt, wurden die HEK 293 Zellen in MEM-Puffer in einer 96-Well-Platte ausgesät und drei Tage lang in einer Atmosphäre von 95 % relativer Luftfeuchtigkeit und 5 % CO₂ gezüchtet. Danach wurde das Medium entfernt, die Zellen wurden mit 20 mM HEPES, 268 mM Glukose, pH 7,4 gewaschen und mit 100 µL ummantelten Nanoteilchen, die 1:1, 1:10 und 1:50 (v/v) in Glukose-HEPES-Puffer verdünnt waren, inkubiert. Zusätzlich wurden 0,1 % Triton-X-Lösung und Glukose-HEPES-Puffer als Positiv- bzw. Negativkontrolle verwendet. Die Platte wurde 24 Stunden lang bei 37°C (150 rpm) inkubiert. Nach zwei, vier und 24 Stunden wurden die Proben entnommen, die Zellen einmal mit Glukose-HEPES-Puffer gewaschen und 100 µL einer in Glukose-HEPES-Puffer hergestellten 0,5 mg/mL MTT-Lösung in jede Vertiefung gegeben. Die Platte wurde 2,5 Stunden lang inkubiert. Danach wurde die MTT-Lösung entfernt und die gebildeten Formazan-Kristalle wurden in 120 µL Dimethylsulfoxid (DMSO) gelöst. 100 µL der resultierenden Lösungen wurden in eine 96-Well-Platte überführt und die Absorption wurde bei einer Wellenlänge von 540 nm mit einem Mikroplattenlesegerät gemessen. Insgesamt wurde bei fast allen untersuchten Nanoteilchen eine zeit- und konzentrationsabhängige Zelltoxizität beobachtet, wie in Fig. 3B dargestellt. Im Einzelnen zeigten mit TPP ummantelte Nanoteilchen bei einer Verdünnung von 1:10 und 1:50 (v/v) die höchste Zellviabilität, gefolgt von PP und PA. Bei der 1:10 Verdünnung (der TPP ummantelten Nanoteilchen) waren nach Inkubation von zwei und vier Stunden etwa 90 % ± SD der HEK 293 Zellen viabel sowie nach der Inkubation von 24 Stunden etwa 55 % ± SD.

### Herstellung und Charakterisierung von mit Wirkstoff beladenen ummantelten Nanoteilchen

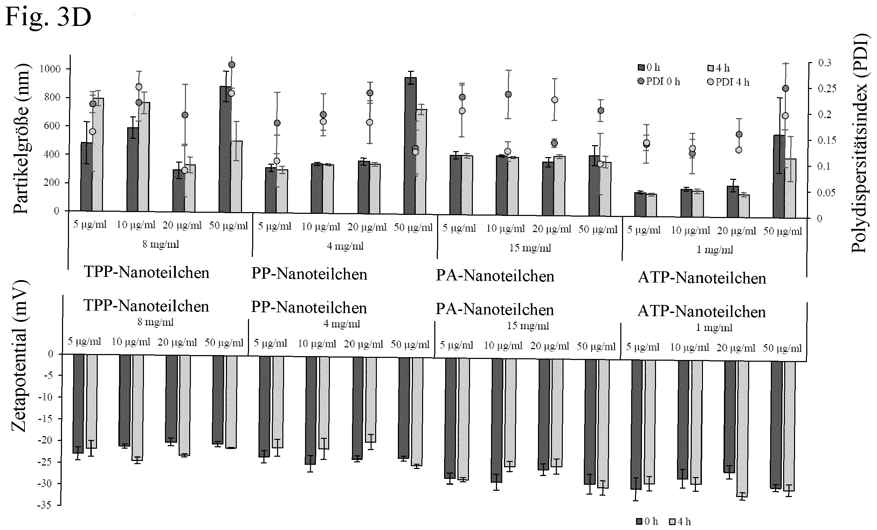

Zudem wurden Untersuchungen zur Herstellung und Charakterisierung von mit Wirkstoff beladenen ummantelten Nanoteilchen durchgeführt (siehe Fig. 3C). Dabei wurde das Antiseptikum Ethacridin-Lactat (ETH) als Modellarzneimittel ausgewählt, da es den Vorteil hat, positiv geladen zu sein (bzw. als Kation vor zu liegen), und sich daher für die Versetzung bzw. das Einbringen (encapsulation) an anionisch ummantelte Nanoteilchen eignet, ähnlich wie die meisten Krebsmedikamente. Ummantelte Nanoteilchen wurden mit unterschiedlichen ETH-Konzentrationen von 5 µg/mL bis 50 µg/mL versetzt. Dabei zeigte sich eine höhere Versetzungs-Effizienz (encapsulation efficiency) für PP und PA, während für TPP-ummantelte Nanoteilchen eine niedrigere Versetzungs-Effizienz und für ATP-ummantelte Nanoteilchen eine geringfügig niedrigere Versetzungs-Effizienz (encapsulation efficiency) beobachtet wurde. Darüber hinaus wurden auch die Partikelgröße, der Polydispersitätsindex (PDI) und das Zetapotenzial des erfindungsgemäßen Gegenstands (Wirkstoff-beladenes ummanteltes Nanoteilchen) untersucht, wobei eine höhere Stabilität für Proben beobachtet wurde, die mit einer Wirkstoffkonzentration von 20 µg/mL versetzt waren und daher für nachfolgende Studien ausgewählt wurden (siehe Fig. 3D).

### Messungen zur Wirkstofffreisetzung

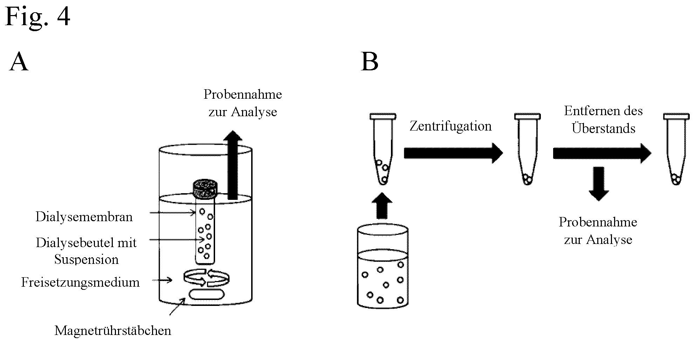

Um den Einfluss der enzymatischen Aktivität auf die Wirkstofffreisetzung von Ethacridin-Lactat (ETH) aus beladenen Nanoteilchen zu bewerten, wurden Studien zur Wirkstofffreisetzung anhand unterschiedlicher Methoden durchgeführt (siehe Fig. 4A und Fig. 4B), welche im Folgenden beschrieben werden.

Zunächst wurden die Wirkstofffreisetzungsprofile bewertet, indem der freigesetzte Wirkstoff physikalisch von den Trägern getrennt wurde, indem eine Dialysemembran mit einem spezifischen Cut-off verwendet wurde, der es dem Wirkstoff ermöglichte, durch die Membran in das Freisetzungsmedium zu gelangen (siehe Fig. 4A). Aus der Lösung außerhalb des Dialysebeutels wurden Proben entnommen und zur Quantifizierung des Wirkstoffs verwendet. Bei der zweiten Methode wurden die Nanoteilchen durch Zentrifugation vom Freisetzungsmedium getrennt und die Menge des freigesetzten Wirkstoffs im Überstand analysiert (siehe Fig. 4B). Auf der Grundlage der Ergebnisse der Charakterisierung der beladenen Träger wurden ummantelte Nanoteilchen gewonnen, die 20 µg/mL ETH enthielten. Die gereinigten ummantelten ETH-haltigen Nanoteilchen, die nach der Zentrifugation gesammelt wurden, wurden in 1 mL demineralisiertem Wasser resuspendiert und vor den Freisetzungsstudien 1:10 (v/v) mit 20 mM HEPES-Puffer verdünnt.

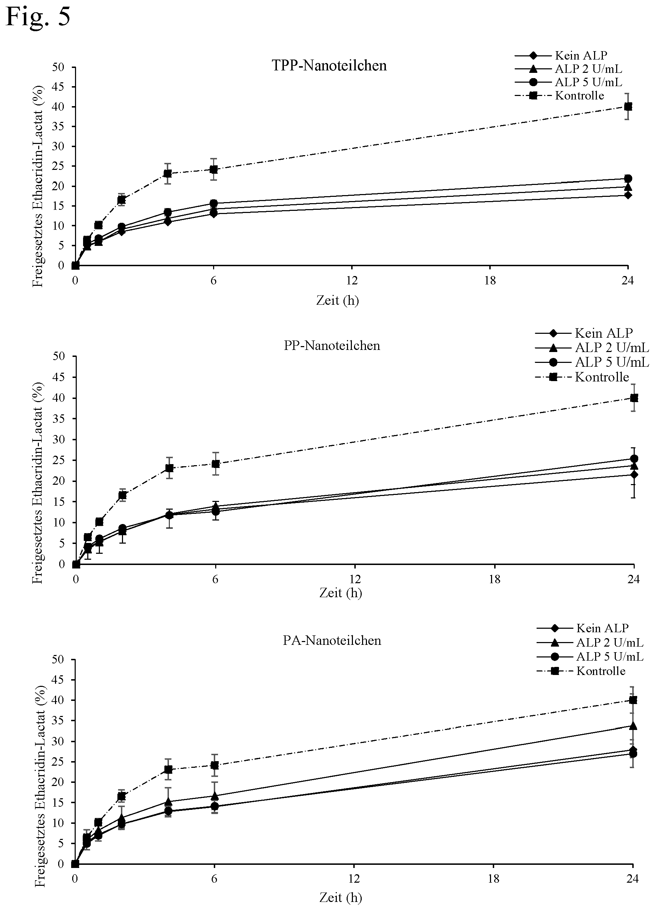

Fig. 5 zeigt erste Untersuchungen zur Wirkstofffreisetzung von mit Wirkstoff beladenen Nanoteilchen über eine Dialysemembran. Dabei wurde die Ummantelung aus TPP, PP oder PA mit einem Wirkstoff versetzt. 20 µg/mL Ethacridin-Lactat (ETH) wurde nach einer Verdünnung von 1:10 (v/v) in 20 mM HEPES-Puffer verwendet. Die Freisetzung von Ethacridin-Lactat (%) wurde nach einer 24-stündigen Inkubation bei 37°C unter Schütteln bei 300 rpm gemessen. Die Freisetzungsversuche wurden mit einem Dialyseschlauch aus regenerierter Cellulose durchgeführt (14 kDa). Die angegebenen Werte sind Mittelwerte von n=3 ± SD. Es konnte eine Wirkstofffreisetzung von zwischen 18 und 33 % ± SD nach 24 Stunden gezeigt werden.

Eine weitere Versuchsreiche, bei der die Freisetzung durch Zentrifugation erfolgte, ergab eine maximale Wirkstofffreisetzung Ethacridin-Lactat (ETH) von bis zu 60 % ± SD nach 48 Stunden (Daten nicht gezeigt). Generell ist zu beachten, dass es sich um eine *in vitro* Situation handelte und kein externes Magnetfeld eingesetzt wurde. Es sei auch darauf hingewiesen, dass die Wahl des Wirkstoffs für die hier dargelegte Versuchsreiche die bereits genannten bevorzugten Wirkstoffklassen nicht ausschließt, wobei die Konzentration von 20 µg/mL auch für die bevorzugten Wirkstoffklassen von Vorteil sein kann.

Fig. 6A zeigt grob schematisiert ein Nanoteilchen 1 gemäß der Erfindung. Das Nanoteilchen 1 zur Verabreichung eines Arzneimittels umfasst einen magnetisierbaren bzw. magnetischen Kern 2, und zwar ist dieser entweder superparamagnetisch (er besteht in den untersuchten Beispielen aus Fe₂O₃ bzw. Fe₃O₄ oder aus Maghemit (*γ*-Fe₂O₃)) oder ferritisch (er besteht in den untersuchten Beispielen aus Cobaltferrit (CoFe₂O₄)) ausgebildet. Der superparamagnetische oder ferritische Kerns 2 wird von einer Ummantelung 3 aus Polyphosphat vollständig umgeben. Die Ummantelung 3 aus einem Polyphosphat ist mit zumindest einem Wirkstoff 4 versetzt, wobei der Wirkstoff 4 an das Polyphosphat ionisch gebunden ist. Als kationische Wirkstoff wurden Zytostatika, Hormone, Antikörper, Fusionsproteine, Peptide, Zytokine, Signaltransduktions-Inhibitoren, Protease-Inhibitoren, HMG-CoA-Reduktase-Inhibitoren, Analgetika und Mischungen daraus untersucht.

In onkologischen Untersuchungen wurden als Wirkstoff Zytostatika verwendet, die - wie in Fig. 6B dargestellt - über eine Spritze 11, einen Trokar, eine Biopsie-Nadel oder einen Katheter lokal oder systemisch in den Körper 10 injiziert wurden. Lokal oder systemisch im Körper 10 verteilen sich die einzelnen Nanoteilchen 1 und werden in der Nähe eines Tumors 12 mittels eines Magnetfels einer Magnetquelle 14 gelenkt und dort gehalten, wo sie den Wirkstoff zielgerichtet an den Tumor 12 abgeben und dort die zytostatische Wirkung entfalten können.

## Patentansprüche

1. Nanoteilchen (1) zur Verabreichung zumindest eines Wirkstoffs (4), umfassend einen magnetisierbaren Kern (2), wobei der Kern (2) ein Eisenoxid umfasst und eine Ummantelung (3) aus einem Polyphosphat aufweist, wobei die Ummantelung (3) den Kern (2) vorzugsweise vollständig umschließt, wobei die Ummantelung (3) mit einem vorzugsweise kationischen Wirkstoff (4) versetzt ist.

2. Nanoteilchen nach Anspruch 1, **dadurch gekennzeichnet, dass** der Wirkstoff (4) an das Polyphosphat ionisch gebunden ist.

3. Nanoteilchen nach Anspruch 1 bis 2, **dadurch gekennzeichnet, dass** der Kern (2) superparamagnetisch oder ferritisch ist.

4. Nanoteilchen nach einem der Ansprüche 1 bis 3, **dadurch gekennzeichnet, dass** die Ummantelung (3) aus Polyphosphat Tripolyphosphat (TPP), Natriumpolyphosphat (PP), Pentapolyphosphat, Hexametaphosphat, Graham's Salz, Phytinsäure (PA), Guanosintriphosphat (GTP), Adenosintriphosphat (ATP) und/oder Mischungen daraus umfasst.

5. Nanoteilchen nach einem der Ansprüche 1 bis 4, **dadurch gekennzeichnet, dass** die Ummantelung (3) zusätzlich zum Polyphosphat zumindest eine Verbindung aus der Gruppe bestehend aus Dextran, Carboxydextran, Carboxymethyldextran, PEG-starch, Siloxane, sulfonierte Styrenedivinylbenzene, Copolymer und Mischungen daraus umfasst.

6. Nanoteilchen nach einem der Ansprüche 1 bis 5, **dadurch gekennzeichnet, dass** das Nanoteilchen eine Gesamtmasse (G1) aufweist, wobei die Gesamtmasse (G1) den Kern (2) und die Ummantelung (3) umfasst, wobei die Gesamtmasse der Ummantelung (3) zumindest 5 %, vorzugsweise zumindest 20 % bezogen auf die Gesamtmasse (G1) beträgt.

7. Nanoteilchen nach einem der Ansprüche 1 bis 6, **dadurch gekennzeichnet, dass** der Wirkstoff (4) ausgewählt ist aus der Gruppe bestehend aus Zytostatikum, Hormon, Antikörper, Fusionsprotein, Peptide, Zytokin, Signaltransduktions-Inhibitor, Protease-Inhibitor, Proteasom-Inhibitor, HMG-CoA-Reduktase-Inhibitor, Analgetikum und Mischungen daraus.

8. Nanoteilchen nach einem der Ansprüche 1 bis 7, **dadurch gekennzeichnet, dass** es in getrockneter Form vorliegt.

9. Nanoteilchen nach einem der Ansprüche 1 bis 8 zur Verwendung als Arzneimittel.

10. Nanoteilchen zur Verwendung nach Anspruch 9 zur Verwendung in der Humanmedizin oder Veterinärmedizin, insbesondere in der Onkologie, Immunologie, Hämatologie und/oder Schmerztherapie.

11. Nanoteilchen zur Verwendung nach Anspruch 9 oder Anspruch 10 für eine modifizierte Freisetzung des Wirkstoffs.

12. Nanoteilchen zur Verwendung nach Anspruch 11, wobei die modifizierte Freisetzung über einen Zeitraum von 5 Minuten bis 8 Stunden erfolgt.

13. Arzneimittel, umfassend ein Nanoteilchen (1) nach einem der Ansprüche 1 bis 8 und einen Wirkstoff (4).

14. Verfahren zur Herstellung eines Nanoteilchens (1) nach einem der Ansprüche 1 bis 12, **dadurch gekennzeichnet,**
(a) **dass** ein magnetisierbarer Kern (2) bereitgestellt wird, wobei der Kern (2) ein Eisenoxid umfasst,
(b) **dass** eine Mischung aus Polyphosphat mit einem Wirkstoff (4) bereitgestellt wird, und
(c) **dass** der magnetisierbare Kern (2) mit der Mischung aus Polyphosphat mit Wirkstoff (4) ummantelt wird, wobei vorzugsweise die nach Schritt (c) erhaltenen Nanoteilchen in einem Schritt (d) getrocknet werden.

15. Kit, umfassend
• Nanoteilchen nach einem der Ansprüche 1 bis 12 in einem Behälter,
• einen physiologisch annehmbaren Puffer in einem getrennten Behälter, sowie
• eine Spritze,
vorzugsweise zur Verwendung in der Zubereitung einer gebrauchsfertigen Suspension aus den Nanoteilchen im Puffer unmittelbar vor der Injektion der Suspension.
